# EUROPEAN PATENT SPECIFICATION

(11) **EP 0 904 726 B1**
(45) Date of publication and mention of the grant of the patent: **24.08.2005**
(21) Application number: 97920487.2
(22) Date of filing: 05.05.1997
(51) Int. Cl.: A61B 1/267

(54) **ANESTHETIC LARYNGOSCOPE WITH MANUAL CONTROLLED OXYGEN EJECTION MEANS**
NARKOSE-LARYNGOSKOP MIT HANDBEDIENTER SAUERSTOFF-ABGABE-VORRICHTUNG
LARYNGOSCOPE ANESTHESIQUE AVEC MOYEN D'EJECTION DE L'OXYGENE A COMMANDE MANUELLE

(30) Priority: 06.05.1996 CN 96106088
(43) Date of publication of application: 31.03.1999
(73) Proprietor: Ha, Da, Neimenggu 010062 (CN)
(72) Inventor: Ha, Da, Neimenggu 010062 (CN)
(74) Representative: Moncheny, Michel
(86) International application number: PCT/CN1997/000040
(87) International publication number: WO 1997/041768

(56) References cited:
- CN-U- 2 046 364
- CN-U- 2 094 963
- CN-U- 2 100 183
- CN-U- 88 203 809
- SU-A- 1 101 220
- US-A- 3 941 120
- US-A- 4 126 127
- US-A- 4 681 094
- US-A- 4 947 896

## Description

### Technical Field

The present invention relates to a laryngoscope used as a medical apparatus. More particularly, the present invention relates to an anesthetic laryngoscope with manual controlled oxygen ejection means.

### Background Art

It is well known in the art that laryngoscope is a tool for using to expose a patient's glottis during an endotracheal intubation operation which is carried out in a field cardiopulmonary resuscitation processing and introduces general anesthesia. Currently, the commonly used methods for opening the patient's air passage in the field cardiopulmonary resuscitation processing are as follows.
(1) Mouth to mouth artificial respiration. Its merit is rapid action. However, it will be easy to exhaust the operator's physical strength during the operation and its effect is not reliable. In addition, this method has a possibility of spreading some diseases in said processing.
(2) Esophagus blocked type of air tube. The use of this method is very convenient and is easy to be popularized. However, asphyxiation due to mishandling it into the patient's trachea in the processing and stomach expansion as its potential complications are presented. Esophagus rupture will be induced when the tube is drawn out before the air-pocket is loosened. As for the patient whose respiratory reflex is not restored, gastric contents will reflow into the air passage when the tube is pulled out so that respiratory tract blocking will be induced. For this reason, tracheal cannula should be applied to the patient prior to pulling out of the tube. Since the facemask is difficult to closely engage the patient's face, hypoventilation and hypercapnia will occur.
(3) Tracheal cannula. After the tracheal cannula is administrated, the air passage may maintain unobstructed so that it can not introduce gastric contents to be inhaled into the air tube erroneously and stomach expansion will not occur. Afterwards, intermittent positive pressure artificial ventilation can be carried out and the foreign materials inside the trachea can also be directly sucked out. Therefore, the method of administrating tracheal cannula is a very reliable method to maintain respiratory tract clear in cardiopulmonary resuscitation processing. The method of administrating tracheal cannula has specific technical difficulty in operating and needs to cost a certain time (preferably not more than 15-20 seconds), meanwhile it is easy to suffer an influence of such as obese body, short neck or higher laryngeal protuberance. Although sometimes the larynx has been exposed, the glottis can not be seen clearly so that it is hardly to insert the cannula. If the sustain time of non-aerated phase lasts too long, the tissue anoxia will become more serious. Thus, the patient will take a turn for the worse so far as to produce emergency.

In order to decrease mortality and increase savage mission success rate, the inventor has disclosed a multifunctional laryngoscope in his prior Chinese Patent No.88203809.5. This invention has made an improvement on the present laryngoscope, in which a tank for storing drugs and a tube for supplying oxygen have been added. The aim of this invention is to provide a laryngoscope which can not only supply oxygen but also administrate atomizing anesthesia in glottis during the operation of administrating tracheal cannula. However, the drawback of this kind of laryngoscope in use lies in that it can not produce pulses when administrating the tracheal cannula during the cardiopulmonary resuscitation processing so that ejection of oxygen can not be achieved. If necessary, an open-type special ejection respirator should be used at the same time. The principle of this apparatus is that the gas flow is blocked by the opening and closing of an electromagnetic valve of the respirator so as to generate pulses, then an oxygen supply tube provided on the laryngoscope blade ejects a supply of oxygen in front of the exposing glottis so that oxygen enters into lungs and the thorax is up and down until it finishes the gas exchange. This open-type special ejection respirator needs to use a storage battery during the field emergency treatment. Therefore, this respirator is bulky and heavy. Thus, its production cost is high and it is not easy to be carried. These drawbacks have virtually limited the flexibility of the laryngoscope in field emergency treatment. Although the open-type special ejection respirator is provided with manual controlled oxygen blocking valve means to prevent the electromagnetic valve from being ineffective, an assistant is required to handle the manual controlled oxygen blocking valve means, thereby it being not convenient to use during the administration of the tracheal cannula. US-A-4 947 896 discloses a laryngoscope, comprising a handle provided with a handle housing and a battery housing, a laryngoscope blade being connected to the handle housing and being provided with an observation light. An oxygen supply tube extends through the handle housing and the laryngoscope blade.

### Summary of the Invention

In order to overcome the above-mentioned drawbacks of the prior art laryngoscopes, an object of the invention is to provide an anesthetic laryngoscope with manual controlled oxygen ejection means which is not restricted by the place of use and can be used to rescue patients without delay. It is small in volume and convenient to use. In addition, it is easy to be produced with lower cost.

To accomplish the object of the invention, there is provided an anesthetic as laryngoscope as claimed in claims 1-5.

These and the other features and advantages of the present invention will become apparent to those skilled in the art upon a reading of the following detailed description when taken in conjunction with the drawings wherein there are shown and described some illustrative embodiments of the invention, which do not mean to limit the protection scope of the invention.

### Brief Description of the Drawings

In the following detailed description, reference will be made to the attached drawings in which:
FIG. 1 is a partially cross sectional view of the anesthetic laryngoscope with manual controlled oxygen ejection means according to the invention;
FIG. 2 is a sectional view of the anesthetic laryngoscope with manual controlled oxygen ejection means according to the invention;
FIG. 3 is a sectional view taken along the line A-A in FIG. 2;
FIG. 4 is a sectional view of the anesthetic laryngoscope with manual controlled oxygen ejection means according to another preferred embodiment of the invention;
FIG. 5 is a detail of the oxygen on/off valve provided in the anesthetic laryngoscope shown in FIG. 4.

### Best Mode for Carrying out the Invention

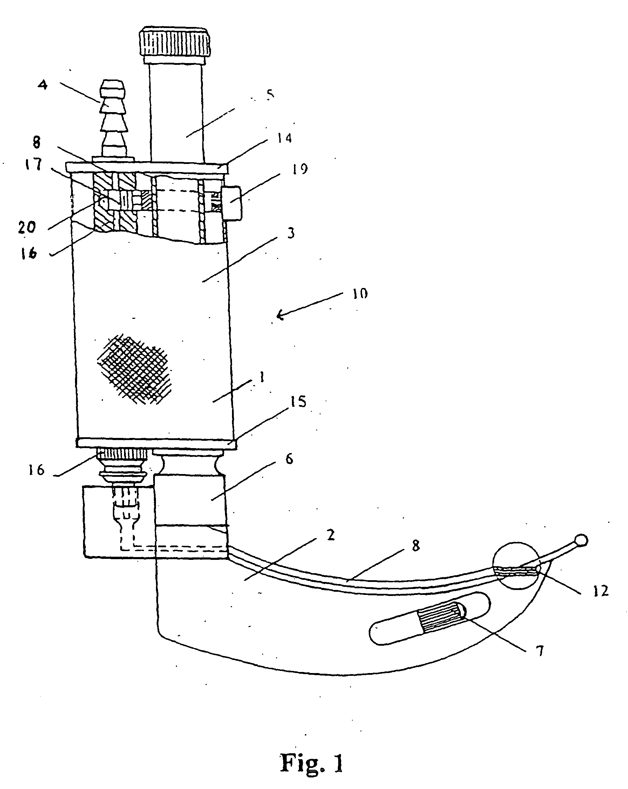

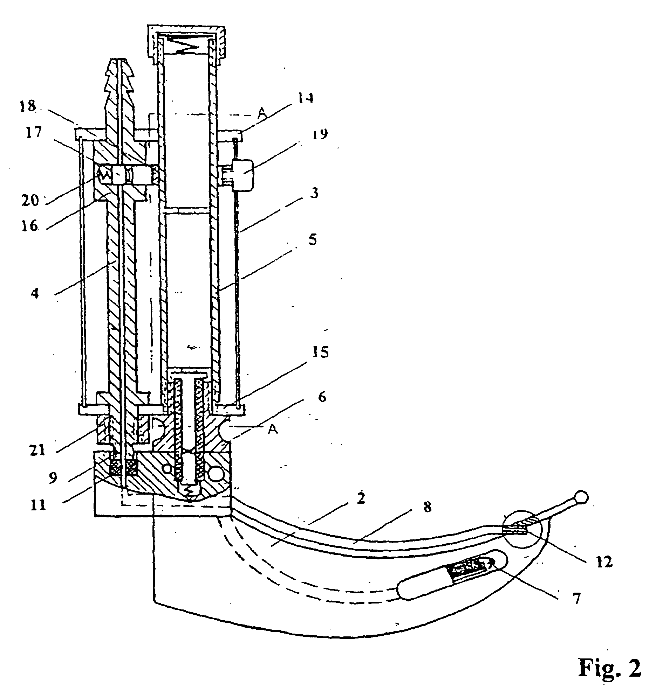

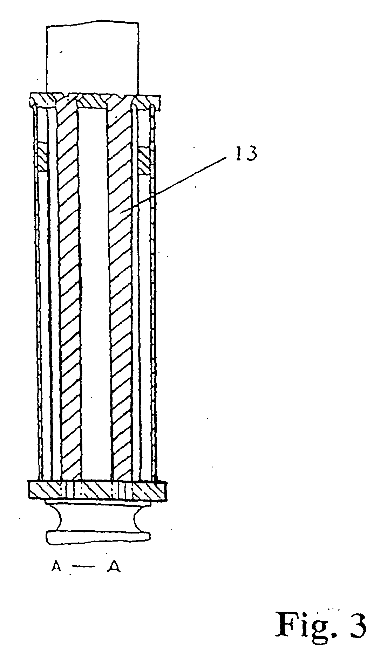

As shown in Figures 1 to 3, the laryngoscope generally designated by the reference numeral 10 comprises a laryngoscope handle 1 and a laryngoscope lens 2. The laryngoscope handle 1 has a laryngoscope handle housing 3 in which a first oxygen supply tube 4 and a battery housing 5 are provided. Moreover, the laryngoscope handle housing 3 is provided between a top cover 14 and a bottom cover 15. A screw stem 13 is also provided between the top cover 14 and the bottom cover 15. A length of thread is provided at the rear end of the first oxygen supply tube 4 on which a nut 21 is threaded. The top cover 14 and the bottom cover 15 are locked together by means of the nut 21. The laryngoscope blade 2 is provided at the bottom end of the laryngoscope handle 1 and is engaged in the socket 6 provided on the bottom end of the laryngoscope handle 1. The laryngoscope blade 2 is provided with an observation light 7 and a second oxygen supply tube 8. A recess 9 is provided at the rear end of the laryngoscope blade 2, in which a gasket 11 is provided so that the second oxygen supply tube 8 of the laryngoscope blade 2 can be engaged and communicated with an oxygen nozzle provided at the bottom end of the first oxygen supply tube 4. Since the mode and the size of the laryngoscope blade 2 will be varied depending on the age of the patient, the interchange of the different laryngoscope blade 2 can be carried out by the engagement of the oxygen supply tubes 4 and 8. The oxygen nozzle 12 of the second oxygen supply tube 8 is provided on the laryngoscope blade 2 at a distance 1 mm-2,5 cm from the leading end of the latter. The diameter of the second oxygen supply tube 8 is about 1-2 mm, preferably 1.63 mm. The nozzle 12 is slight converged and its diameter is about 0.6-0.8 mm.

The laryngoscope handle 1 is provided with a manual controlled oxygen on/off valve which comprises a valve body 16, a valve core 17 and a valve rod 18. The valve body 16 of the manual controlled oxygen on/off valve is mounted on the first oxygen supply tube 4. The valve rod 18 is connected with the control knob 19 and bypasses the battery housing 5 to control the valve core 17 of the manual controlled oxygen on/off valve. A return spring 20 is provided at the end of the valve core 17 of the manual controlled oxygen on/off valve.

The manual controlled oxygen on/off valve can be provided theoretically in any position of the oxygen supply tube, preferably on the laryngoscope handle in view of the practice, so that it is convenient to be handled.

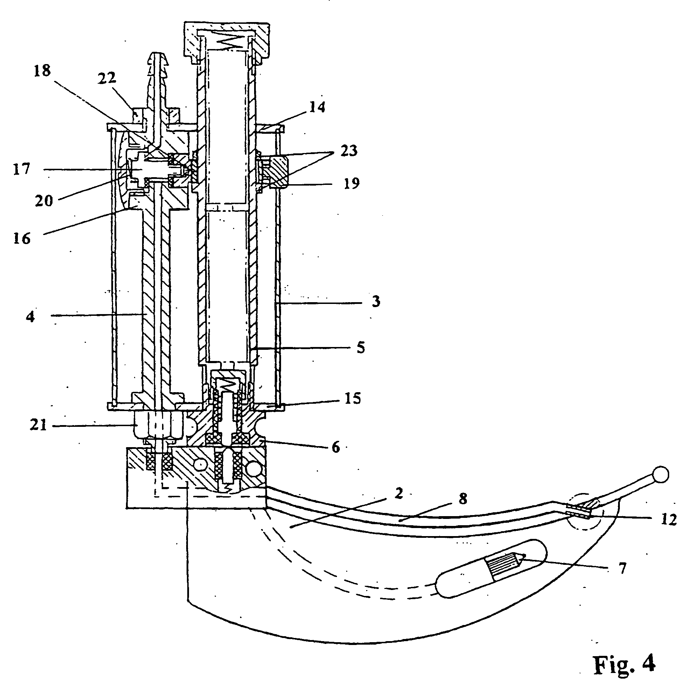

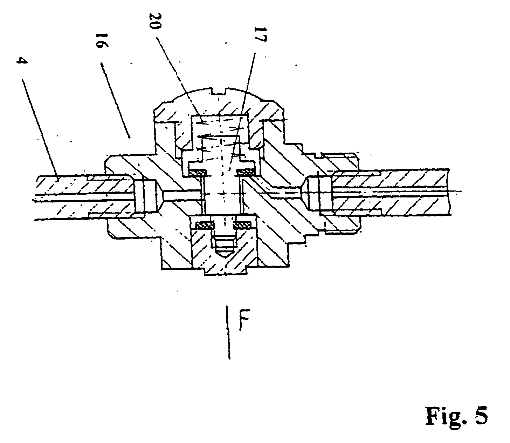

In Figure 4, another preferred embodiment of the anesthetic laryngoscope with manual controlled oxygen ejection means according to the invention is shown, wherein the corresponding components are designated by the same reference number as that of the above-described embodiment, respectively. The difference between the embodiments shown in Figures 1-3 and Figure 4 lies in that the laryngoscope in Figure 4 is provided with another kind of two-position and two-way oxygen valve which is shown in detail in Figure 5 and has more excellent seal effect. In addition, the connection of the laryngoscope handle housing 3 to the top cover 14 and the bottom cover 15 is modified. More specifically, the laryngoscope handle housing 3 is fixed between the top cover 14 and the bottom cover 15 via an upper and lower nuts 21, 22 provided on the first oxygen supply tube 4. The advantage of this arrangement is that the action of the valve rod of the oxygen valve is easier. Furthermore, on the outer wall of the battery housing, at a position corresponding to that of the oxygen valve, a guide means 23 in the form of an upper and lower flanges is provided so as to facilitate to the more properly directional action of the valve rod. It is obvious to one skilled in the art that other types of valves can be adopted to control the oxygen passing through the oxygen supply tube and the connection of the laryngoscope handle housing 3 to the top cover and the bottom cover can be modified in the other proper manner.

The laryngoscope according to the invention can be used together with a small pressure adjustable oxygen bottle. Thus, the laryngoscope of the invention is portable and can be carried with various appendages, such as simplified respirator, manually operated aspirator, tracheal catheter, bit-block, etc.

When rescuing a patient, firstly, an operator connects an oxygen supply source to the laryngoscope according to the invention while adjusting the oxygen pressure, that is, for adult, the pressure is set to 0.12-0.25 MPa; for children, 0.08-0.1 MPa; for neonatus, 0.04-0.06 MPa. After that, the operator holds the laryngoscope 10 by his left hand and makes the angle formed between the laryngoscope handle 1 and the laryngoscope blade 2 to be 90 °. At the same time, the electric source and the gas source are switched on, respectively. After the patient's head position is adjusted, the operator opens the patient's mouth and elevates his lower jaw by using the thumb, fore-finger and middle finger of his right hand while sweeping the patient's labium inferius. When the laryngoscope is put into the patient's mouth along the left side of the right corner of his mouth, the patient's tongue is pushed to the left side and the laryngoscope blade 2 is moved in the center of the patient's mouth. Here, the patient's uvula can be seen, which is the first mark of exposing the patient's glottis. Then, the laryngoscope is advanced slowly until its top end reaches the root of the patient's tongue. After the laryngoscope is lifted up slightly, the border of the patient's epiglottis can be seen, which is the second mark of exposing the patient's glottis. At this moment, the control knob 19 provided on the laryngoscope handle of the present invention is pushed down by the fore-finger of the operator's left hand so that the pulses of the gas flow of oxygen can be created. Thus, the ejection of oxygen in front of the patient's glottis can be carried out while the glottis is exposing. Even if the glottis of the patient can not be seen clearly while the end of larynx has been exposed because of the patient being obese body, short neck or higher laryngeal protuberance, the gas flow can pass through the glottis into the trachea so as to make the gas exchange.

It should be understood, however, that even though the purposes of exemplification and numerous characteristics of the present invention have been set forth in the foregoing description, the disclosure is illustrative only, and modifications and variations to the embodiments of the invention described above may be made by persons skilled in the art without departing from the protection scope of the invention defined by the appended claims.

### Exploitation in Industry

The laryngoscope according to the invention has dispelled the non-aerated phase during the administration of the tracheal cannula and has increased the safety, reliability and the success rate of emergency treatment for the method of administrating tracheal cannula. It has the following advantages: rapidly opening the air passage in the processing so that the supply of oxygen can be restored. The manual controlled frequency can be selected at 12-35 times/min. In the case that food reflow from the stomach occurs in the emergency course, the operator may suck out food while supplying oxygen by using the intubation under direct vision, so that sucking food out and supplying with oxygen can be carried out alternatively. The laryngoscope according to the invention is very useful and better than the other previous ones, particularly for saving the neonatus.

## Claims

1. An anaesthetic laryngoscope (10) with manually controlled oxygen ejection means, comprising
- a laryngoscope handle (1) provided with a laryngoscope handle housing (3) having a lower end, a first oxygen supply tube (4) and a battery housing (5);
- the anaesthetic laryngoscope further comprising a laryngoscope blade (2) provided at said lower end, said laryngoscope blade having an observation light (7) and a second oxygen supply tube (8), said first oxygen supply tube (4) being connected to said second oxygen supply tube (8);
- wherein a manually controlled oxygen on/off valve (16, 17, 18, 19, 20) is provided on one of said first and second oxygen supply tubes (4, 8).

2. Anesthetic laryngoscope according to claim 1, wherein said manually controlled oxygen on/off valve (16, 17, 18, 19, 20) is provided on said first oxygen supply tube (4).

3. Anesthetic laryngoscope according to claim 1, wherein said manually controlled oxygen on/off valve (16, 17, 18, 19, 20) provided on one of said first and second oxygen supply tubes (4, 8) is a manually controlled two position-two way valve.

4. Anesthetic laryngoscope according to claim 1, wherein the diameter of said second oxygen supply tube (8) is in the range of about 1 mm to 2 mm; the second oxygen supply tube (8) has an oxygen nozzle (12) which is at a distance between about 1 mm to about 2.5 cm from the tip of the laryngoscope blade (2) and which is slightly converged, and the diameter of said oxygen nozzle (12) is in the range of about 0.6 mm to 0.8 mm.

5. Anesthetic laryngoscope according to claim 1, wherein a guide means (23) is provided on an outer wall of said battery housing (5) at a position corresponding to that of said valve (16, 17, 18, 19, 20).

## Patentansprüche

1. Anästhesielaryngoskop (10) mit manuell gesteuertem Sauerstoffausstoßmittel, umfassend:
einen Laryngoskopgriff (1), der mit einem Laryngoskopgriffgehäuse (3) mit einem unteren Ende, einem ersten Sauerstoffzufuhrrohr (4) und einem Batteriegehäuse (5) versehen ist;
wobei das Anästhesielaryngoskop ferner ein Laryngoskopblatt (2) umfaßt, das an dem unteren Ende vorgesehen ist, wobei das Laryngoskopblatt ein Beobachtungslicht (7) und ein zweites Sauerstoffzufuhrrohr (8) aufweist, wobei das erste Sauerstoffzufuhrrohr (4) mit dem zweiten Sauerstoffzufuhrrohr (8) verbunden ist;
wobei ein manuell gesteuertes Sauerstoff-Ein-Aus-Schaltventil (16, 17, 18, 19, 20) an einem der ersten und zweiten Sauerstoffzufuhrrohre (4, 8) vorgesehen ist.

2. Anästhesielaryngoskop nach Anspruch 1, wobei das manuell gesteuerte Sauerstoff-Ein-Aus-Schaltventil (16, 17, 18, 19, 20) an dem ersten Sauerstoffzufuhrrohr (4) vorgesehen ist.

3. Anästhesielaryngoskop nach Anspruch 1, wobei das manuell gesteuerte Sauerstoff-Ein-Aus-Schaltventil (16, 17, 18, 19, 20), das an einem der ersten und zweiten Sauerstoffzufuhrrohre (4, 8)) vorgesehen ist, ein manuell gesteuertes Zweiwegeventil mit zwei Positionen ist.

4. Anästhesielaryngoskop nach Anspruch 1, wobei der Durchmesser des zweiten Sauerstoffzufuhrrohrs (8) im Bereich von ungefähr 1 mm bis 2 mm liegt, das zweite Sauerstoffzufuhrrohr (8) eine Sauerstoffdüse (12) aufweist, die sich in einem Abstand von ungefähr 1 mm bis ungefähr 2,5 mm von der Spitze des Laryngoskopblatts (2) befindet und leicht konvergiert, und der Durchmesser der Sauerstoffdüse (12) im Bereich von ungefähr 0,3 mm bis 0,8 mm liegt.

5. Anästhesielaryngoskop nach Anspruch 1, wobei ein Führungsmittel (23) an einer Außenwand des Batteriegehäuses (5) an einer Position vorgesehen ist, die der des Ventils (16, 17, 18, 19, 20) entspricht.

## Revendications

1. Laryngoscope anesthésique (10) avec des moyens d'éjection d'oxygène à commande manuelle, comprenant :
- un manche de laryngoscope (1) doté d'un boîtier formant manche de laryngoscope (3) comportant une extrémité inférieure, un premier conduit d'approvisionnement en oxygène (4) et un boîtier pour batterie (5) ;
- le laryngoscope anesthésique comprenant en outre une lame de laryngoscope (2) située au niveau de ladite extrémité inférieure, ladite lame de laryngoscope comportant une lumière d'observation (7) et un second conduit d'approvisionnement en oxygène (8), ledit premier conduit d'approvisionnement en oxygène (4) étant relié au dit second conduit d'approvisionnement en oxygène (8) ;
- dans lequel une valve marche/arrêt d'approvisionnement en oxygène à commande manuelle (16, 17, 18, 19, 20) est prévue sur l'un desdits premier et second conduits d'approvisionnement en oxygène (4, 8).

2. Laryngoscope anesthésique selon la revendication 1, dans lequel ladite valve marche/arrêt d'approvisionnement en oxygène à commande manuelle (16, 17, 18, 19, 20) est prévue sur ledit premier conduit d'approvisionnement en oxygène (4).

3. Laryngoscope anesthésique selon la revendication 1, dans lequel ladite valve marche/arrêt d'approvisionnement en oxygène à commande manuelle (16, 17, 18, 19, 20) prévue sur l'un desdits premier et second conduits d'approvisionnement en oxygène (4, 8) est une valve à commande manuelle à deux positions-deux voies.

4. Laryngoscope anesthésique selon la revendication 1, dans lequel le diamètre dudit second conduit d'approvisionnement en oxygène (8) est situé dans la gamme comprise entre environ 1 mm et 2 mm ; le second conduit d'approvisionnement en oxygène (8) comporte une buse à oxygène (12) qui est située à une distance comprise entre environ 1 mm et environ 2,5 cm de l'extrémité de la lame de laryngoscope (2) et qui est légèrement convergente, et le diamètre de ladite buse à oxygène (12) est situé dans la gamme comprise entre environ 0,6 mm et 0,8 mm.

5. Laryngoscope anesthésique selon la revendication 1, dans lequel un moyen de guidage (23) est prévu sur une paroi extérieure dudit boîtier pour batterie (5) et est situé en un emplacement correspondant à celui de ladite valve (16, 17, 18, 19, 20).
